(19) Europäisches Patentamt
European Patent Office
Office européen des brevets

(11) Veröffentlichungsnummer: **0 211 205**
**B1**

# (12) EUROPÄISCHE PATENTSCHRIFT

(45) Veröffentlichungstag der Patentschrift: **31.08.88**

(51) Int. Cl.⁴: **C 07 C 45/67**, C 07 C 47/21, C 07 C 47/232

(21) Anmeldenummer: **86108498.6**

(22) Anmeldetag: **21.06.86**

(54) **Verfahren zur Herstellung von 2-Methyl-2-alkenalen.**

(30) Priorität: **28.06.85 DE 3523181**

(43) Veröffentlichungstag der Anmeldung: **25.02.87 Patentblatt 87/9**

(45) Bekanntmachung des Hinweises auf die Patenterteilung: **31.08.88 Patentblatt 88/35**

(84) Benannte Vertragsstaaten: **BE CH DE FR GB IT LI NL**

(56) Entgegenhaltungen:
**EP-A-0 008 022**
**EP-A-0 171 216**
**FR-A-2 028 390**
**US-A-4 417 089**

(73) Patentinhaber: **BASF Aktiengesellschaft, Carl-Bosch- Strasse 38, D-6700 Ludwigshafen (DE)**

(72) Erfinder: **Hupfer, Leopold, Dr., Waltershoehe 3, D-6701 Friedelsheim (DE)**
Erfinder: **Merger, Franz, Dr., Max- Slevogt- Strasse 25, D-6710 Frankenthal (DE)**
Erfinder: **Broecker, Franz Josef, Schwanthaler Allee 20, D-6700 Ludwigshafen (DE)**
Erfinder: **Fischer, Rolf, Dr., Bergstrasse 98, D-6900 Heidelberg (DE)**

**EP 0 211 205 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein Verfahren zur Herstellung von 2-Methyl-2-alkenalen durch Isomerisierung von 2-Alkylacroleinen in Gegenwart von Wasserstoff und von Katalysatoren, die als aktive Bestandteile Palladium und Verbindungen der Seltenen Erdmetalle enthalten.

Bekanntlich lassen sich 2-Alkylacroleine, wie i-Propyl- oder sek.-Butylacrolein mit Wasserstoff in Gegenwart von Palladium auf Aktivkohle als Träger bei 100°C und 20 bar zu den entsprechenden gesättigten Aldehyden hydrieren (DE-OS-2 917 779, Beispiel 1b, DE-OS-2 933 919, Beispiel 1c):

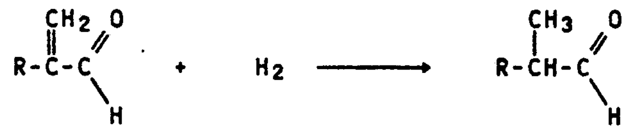

(R = i-Propyl, sek.-Butyl)

Es ist weiterhin bekannt, daß sich 2-Alkyl-2-alkenale, wie 2-Ethyl-2-hexenal mit Wasserstoff in Gegenwart von Palladium auf $SiO_2$ bei 90°C und 200 bar zu 2-Ethylhexanal umsetzen lassen (DE-OS-1 941 634, Beispiel 1):

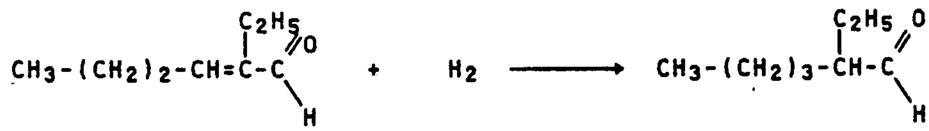

Anstelle von Palladium-Katalysatoren lassen sich bei dieser Hydrierung besonders vorteilhaft solche Tragerkatalysatoren einsetzen, die neben Palladium zusätzlich Oxide von Seltenen Erden, wie Praseodymoxid oder Cerdioxid enthalten (EP-PS 8 022). In der DE-OS-2 621 224, Beispiel 11) wird angegeben, daß sich Acetoxyethylacrolein in Gegenwart von Katalysatoren, die aus Palladium (5 Gew.-%) Tetramethylthioharnstoff und Kohle bestehen, und von Wasserstoff bei 100°C und Normaldruck in Xylol als Lösungsmittel zu 2-Methyl-4-acetoxy-2-butenal isomerisieren läßt.

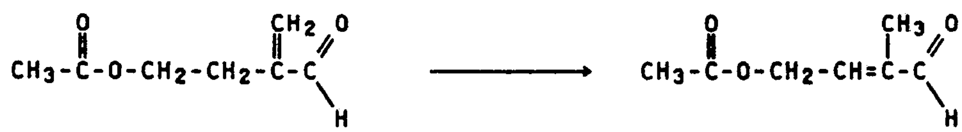

Es bestand die Aufgabe, ein Verfahren zu entwickeln, das die Isomerisierung der aus Alkanalen und Formaldehyd leicht zugänglichen 2-Alkyl-acroleine zu 2-Methyl-2-alkenalen gestattet.

Es wurde nun gefunden, daß sich 2-Methyl-2-alkenale der Formel

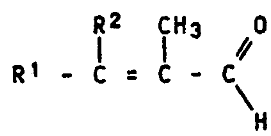

I,

in der $R^1$ und $R^2$ Wasserstoffatome, Alkylreste mit 1 bis 18 Kohlenstoffatomen, die zusätzlich aromatische Reste tragen können, oder aromatische Reste bedeuten, dadurch besonders vorteilhaft herstellen lassen, daß man 2-Alkylacroleine der Formel

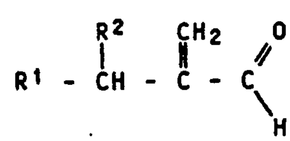

II,

in der $R^1$ und $R^2$ die obengenannte Bedeutung besitzen, in Gegenwart von Katalysatoren, die als aktive Bestandteile Palladium und ein Oxid oder Salz eines Seltenen Erdmetalls enthalten, bei Temperaturen von 20 bis 120°C, insbesondere 50 bis 100°C und Drücken von 1 bis 100 bar, insbesondere 1 bis 20 bar mit Wasserstoff behandelt.

Die Entstehung von 2-Methyl-2-alkenalen der Formel I als Hauptprodukte des erfindungsgemäßen Verfahrens ist überraschend, da bekanntlich sowohl die 2-Alkylacroleine der Formel II als auch die durch Isomerisierung als Reaktionsprodukte erwünschten 2-Methyl-2-alkenale der Formel I zu den gesättigten Alkanalen hydriert werden können. Es war nicht vorauszusehen, daß unter den Bedingungen des erfindungsgemäßen Verfahrens die Isomerisierung mit ausreichender Geschwindigkeit, die Hydrierung dagegen in erheblich geringerem Maße ablaufen würde.

Das neue Verfahren läßt sich am Beispiel der Isomerisieung von 2-Ethyl-acrolein zu Tiglinaldehyd (Trans-2-methyl-2-butenal) durch die folgende Formelgleichung darstellen:

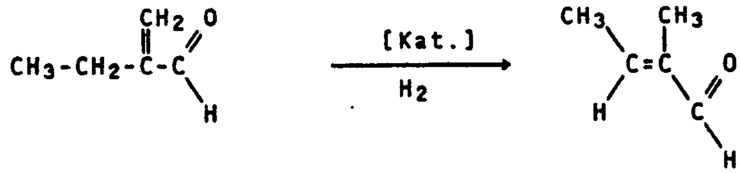


Als Nebenprodukt tritt 2-Methylbutanal auf, das durch Hydrierung von 2-Ethylacrolein und Tiglinaldehyd entsteht.

Die als Ausgangsstoffe verwendeten Acroleinderivate der Formel II enthalten als Reste $R^1$ und $R^2$ Wasserstoffatome, Alkylreste mit 1 bis 18, vorzugsweise 1 bis 12 C-Atomen, die durch aromatische Reste substituiert sein können, oder aromatische Reste. Alkylreste sind z. B. Methyl-, Ethyl-, n-Propyl- i-Propyl-, n-Butyl-, i-Butyl-, tert.-Butyl-, Pentyl-, Hexyl-, Nonyl-, Decyl- oder Dodecylreste. Als aromatische Reste kommen z. B. Phenylreste in Frage, die noch Substituenten, wie Alkyl- oder Alkoxyreste oder Halogenatome enthalten können. Beispielsweise seien die folgenden Verbindungen der Formel II genannt:

2-Ethylacrolein, 2-n-Butylacrolein, 2-i-Propylacrolein, 2-n-Propylacrolein, 2-Decylacrolein, 2-n-Pentylacrolein, 2-Benzylacrolein, 2-Heptylacrolein, 2-n-Hexylacrolein, 2-i-Butylacrolein, 2-n-Nonylacrolein. Die Ausgangsverbindungen der Formel II lassen sich z. B. durch Umsetzung von Alkanalen mit Formaldehyd und sekundären Aminen in Gegenwart von Carbonsäuren herstellen (DE-OS-3 106 557).

Die Katalysatoren, in deren Gegenwart man die erfindungsgemäße Umsetzung vornimmt, enthalten als aktive Bestandteile Palladium oder Platin und ein Oxid oder Salz eines Seltenen Erdmetalles. Beispielsweise kommen als Katalysatoren die in EP 8 022 beschriebenen Katalysatoren in Betracht, deren aktive Bestandteile aus 2 bis 90 Gew.-% Palladium und 10 bis 98 Gew.-% eines Oxids oder eines Salzes eines Seltenen Erdmetalls oder einer Hischung verschiedener derartiger Oxide und/oder Salze bestehen. Neben Palladium sind als katalysatoraktive Zusätze alle Oxide und Salze der Seltenen Erdmetalle (im folgenden als SE-Verbindungen bezeichnet) geeignet. Das sind vornehmlich die Oxide, und zwar insbesondere Lanthanoxid ($La_2O_3$) Samariumoxid ($Sm_2O_3$) Gadoliniumoxid ($Gd_2O_3$) und Holmiumoxid ($Ho_2O_3$) sowie vor allem Ceroxid ($CeO_2$), Praseodymoxid ($Pr_2O_3$) und Neodymoxid ($Nd_2O_3$).

Anstelle der Oxide lassen sich aber auch die Salze der Seltenen Erdmetalle, beispielsweise die Nitrate, Sulfate, Phosphate, Chloride und Carbonate verwenden. Bevorzugt werden Toluolsulfonate und besonders die organischen Salze der höheren Fettsäuren, z. B. Stearinsäure. Sind die Salze in der zu hydrierenden Ausgangsverbindung löslich, so können sie dieser auch vor der Reaktion beigemischt werden, so daß die Mischung nur noch über einen Palladiumkontakt geleitet zu werden braucht. Diese Verfahrensweise empfiehlt sich besonders in den Fallen, in denen man die Hydrierung an einem bereits bestehenden herkömmlichen Palladiumkontakt durchführen möchte. Bei dieser Umstellung auf die erfindungsgemäße Isomerisierung entfällt der Aufwand eines Katalysatorwechsels. Das Verfahrensprodukt braucht dann von dem SE-Salz nur noch abdestilliert zu werden.

Im übrigen ist es nicht erforderlich, die reinen SE-Verbindungen zu verwenden, vielmehr eignen sich ebensogut deren Gemische, z. B. die handelsüblichen Oxide und Salze von technischer Reinheit, die z. B. nur zu 90 Gew.-% aus der reinen SE-Verbindung bestehen, wobei der Rest mehrere, begleitende andere SE-Verbindungen enthalten kann.

Da es bei dem erfindungsgemäßen Verfahren auf die gleichzeitige Gegenwart der beiden Komponenten des Katalysators, nämlich das Palladium einerseits und die SE-Verbindung andererseits ankommt, stellt sich die erfindungsgemäß gewünschte Wirkung bereits dann ein, wenn das Palladium und die SE-Verbindung gemeinsam in einer Suspension des zu hydrierenden Aldehyds oder einer organischen Lösung dieses Aldehyds vorliegen. Ebenso verhält es sich, wenn es sich z. B. um eine Suspension eines Pd-Trägerkatalysators - z. B. mit Aktivkohle als Träger - und eines SE-Oxid-Trägerkatalysators - z. B. mit Aluminiumoxid als Träger - handelt.

Derartige Verfahrensweisen sind prinzipiell möglich und häufig auch für Hydrierungen im Labormaßstab oder im halbtechnischen Maßstab geeignet. Für den kontinuierlichen technischen Betrieb empfiehlt es sich jedoch aus allgemein bekannten verfahrenstechnischen Gründen, den Katalysator in einer Reaktorsäule fest anzuordnen und Wasserstoff und Aldehyd bzw. eine Lösung des Aldehydes über ein derartiges Festbett zu leiten.

Für diesen Zweck verwendet man vorzugsweise Trägerkatalysatoren, auf welchen das Palladium und die SE-Verbindung gemeinsam aufgebracht ist. Man kann solche Trägerkatalysatoren herstellen, indem man das Trägermaterial mit einer wäßrigen Lösung, die ein Pd-Salz, wie Palladiumnitrat sowie ein SE-Salz im entsprechenden Mengenverhältnis enthält, imprägniert, trocknet und im Luftstrom erhitzt, wobei sich das SE-Oxid bildet. Das Pd-Metall bildet sich dann unter den hydrierenden Bedingungen von selber, jedoch kann man auch den Trägerkatalysator zu diesem Zweck einer gesonderten Hydrierung unterwerfen. Als Trägermaterialien eignen sich z. B. Aktivkohle, Aluminiumoxid und Kieselgel in Form von Tabletten, Granalien, Kugeln und Strängen mit Durchmessern von 2 bis 20 mm und einer Längenausdehnung von 5 bis 50 mm. Eine Schüttung von 1 Liter derartiger Trägerkatalysatoren enthält je nach Raumform und Gesamtoberfläche des Trägers etwa 4 bis 80 g aktiver Katalysatorbestandteile. Im allgemeinen beträgt das Gewichtsverhältnis von Pd zur SE-Verbindung 2 : 98 bis 90 : 10, jedoch sind in aller Regel solche Katalysatoren zu bevorzugen, in denen dieses

Verhältnis zwischen 20 : 80 und 80 : 20 liegt.

Die Isomerisierung läßt sich diskontinuierlich oder kontinuierlich als Festbettreaktion, beispielsweise in Sumpf- oder Rieselfahrweise oder aber mit suspendierten Katalysatoren durchführen. Die Reaktionstemperaturen liegen im Bereich von 20°C bis 120°C, insbesondere bei 50 bis 100°C. Die hierbei benötigten Drucke liegen bei Normaldruck bis 100 bar, insbesondere bei 1 bis 20 bar. Im allgemeinen wird man zur Erreichung eines hohen Isomerisierungsanteils und einer ausreichenden Reaktionsgeschwindigkeit einer hohen Reaktionstemperatur einen niedrigeren Druck, einer tieferen Reaktionstemperatur jedoch einen höheren Reaktionsdruck zuordnen.

Bei einer bevorzugten Ausführungsform des Verfahrens führt man die Isomerisierung in einem Lösungsmittel durch, das unter den Reaktionsbedingungen inert ist. Derartige Lösungsmittel sind beispielsweise $C_5$- bis $C_8$-Paraffine, cyclische Kohlenwasserstoffe, wie Cyclohexan, Methylcyclohexan, Toluol, Xylole, Alkohole, wie Methanol oder Ethanol, cyclische Ether wie Tetrahydrofuran oder Dioxan, Ester wie Methyl- oder Ethylacetat. Die Menge des Lösungsmittels ist nicht kritisch und kann z. B. das 0,5- bis 10-fache der Gewichtsmenge des Aldehyds betragen.

Das erfindungsgemäße Verfahren besitzt gegenüber den bekannten Verfahren zur Herstellung von 2-Methyl-2-alkenalen Vorteile. So erzielt man besonders bei der Synthese von 2-Methyl-2-alkenalen mit ungerader Kohlenstoffzahl durch Aldolkondensation zweier verschiedener Aldehyde nur niedrige Ausbeuten. Die Synthese von Tiglinaldehyd aus Acetaldehyd und Propionaldehyd gelingt z. B. nur mit 30 Ziger Ausbeute (Journal für Praktische Chemie, Band 155, Seiten 310 bis 316). Demgegenüber lassen sich Alkanale mit Formaldehyd in hohen Ausbeuten zu 2-Alkylacroleinen umsetzen (DE-OS-3 106 557), die dann nach dem erfindungsgemäßen Verfahren zu 2-Methyl-2-alkenalen isomerisiert werden können. Die beim erfindungsgemäßen Verfahren als Nebenprodukte anfallenden 2-Methylalkanale stellen ebenfalls wertvolle Zwischenprodukte dar, die zu den entsprechenden Carbonsäuren oxidierbar sind.

Die nach dem erfindungsgemäßen Verfahren erhältlichen 2-Methyl-2-alkenale (I) stellen wertvolle Zwischenprodukte für die Herstellung von Farbstoffen, Pharmazeutika und Pflanzenschutzmitteln dar. Sie finden ferner als Aromastoffe Verwendung (DE-PS-2 927 090).

**Beispiel 1**

25 g eines Palladium und Cer enthaltenden Katalysators (0,08 % PdO, 0,61 % $CeO_2$ auf 4 mm $\gamma$-$Al_2O_3$-Strängen), gemischt mit 5 mm Glasringen, wurden in ein senkrecht stehendes gläsernes Reaktorrohr (150 ml) gefüllt und 16 Stunden lang bei 180°C mit Wasserstoff (ca. 3 l $H_2$/Stunde) aktiviert.

Eine Lösung von 60 g 2-Ethylacrolein in 240 g Dioxan wurde in Rieselfahrweise bei 100 $\pm$ 20°C über den Katalysator gepumpt. Die Lösung wurde in einem mit Intensivkühler versehenen 500-ml-Kolben aufgefangen und von dort zurück über den Katalysator geleitet (Umlaufmenge ca. 20 Liter/Stunde). Sechs Liter Wasserstoff wurden stündlich am oberen Ende des mit Katalysator gefüllten Reaktorrohres eingeleitet. Abgas wurde über den wassergekühlten Intensivkühler abgeführt. Etwa alle zwei Stunden wurden dem Reaktionsgemisch Proben entnommen, die gaschromatographisch auf ihren Gehalt an eingesetztem Ethylacrolein, gebildetem Tiglinaldehyd (trans-2-Methyl-2-butenal) und 2-Methylbutanal untersucht wurden (Tabelle 1):

**Tab. 1**

Tiglinaldehyd durch Isomerisierung von Ethylacrolein an Pd/Ce/$Al_2O_3$-Katalysatoren

| Reaktions-zeit [h] | GC-Analyse (Flächen-%)[1] 2-Ethyl-acrolein | Tiglin-aldehyd | 2-Methyl-butanal | Dimeres[2] 2-Ethyl-acrolein |
|---|---|---|---|---|
| 0,1 | 91,8 | 3,1 | 0,6 | 1,4 |
| 2 | 57,4 | 26,6 | 8,0 | 1,4 |
| 4 | 27,8 | 51,3 | 16,0 | 1,7 |
| 5,5 | 10,1 | 62,7 | 20,8 | 1,6 |
| 6,5 | 2,7 | 67,3 | 24,1 | 1,4 |

1 Säule Carbowax 20 M, 4 m, 4 Minuten isotherm 100°C, dann Temperaturprogramm 100 bis 200°C/10°C pro Minute.

2 2,5-Diethyl-2-formyl-2,3-dihydro-4H-pyran.

Nachdem nach 6,5 Stunden praktisch das gesamte Ethylacrolein umgesetzt war, wurde der Reaktionsaustrag (255 g) destillativ aufgearbeitet (Drehbandkolonne). In den zunächst abdestillierten Gemischen aus Dioxan, Ethylacrolein und 2-Methylbutanal wurden durch quantitative GC-Analyse zunächst 1,1 g Ethylacrolein (2 % bez. eingesetztes Ethylacrolein) und 13,3 g 2-Methylbutanal (22 % bez. eingesetztes Ethylacrolein) gefunden.

Zuletzt wurden 28,2 g Tiglinaldehyd (47 % bez. eingesetztes Ethylcrolein) vom Siedepunkt 114 bis 116°C/1000 mbar erhalten. Im Destillationsrückstand (5,5 g Öl) war laut GC-Analyse 2,5-Diethyl-2-formyl-2,3-dihydro-4H-pyran enthalten.

**Beispiel 2**

Wie in Beispiel 1 beschrieben, wurden 125 g $Pd/Ce/Al_2O_3$-Katalysator aktiviert und zunächst 3 Stunden lang bei 50 ± 2°C mit 270 g Ethylacrolein umgesetzt. Sechs Liter Wasserstoff wurden pro Stunde eingeleitet. Tabelle 2 zeigt die Konzentrationsänderungen im Reaktionsgemisch in Abhängigkeit von der Reaktionszeit.

**Tab. 2**

Tiglinaldehyd durch Isomerisierung von Ethylacrolein an $Pd/Ce/Al_2O_3$-Katalysatoren

| | GC-Analyse (Flächen-%) | | | |
|---|---|---|---|---|
| Reaktions-zeit [h] | 2-Ethyl-acrolein | Tiglin-aldehyd | 2-Methyl-butanal | Dimeres 2-Ethyl-acrolein |
| 0,1 | 81,1 | 1,6 | 2,2 | |
| 1 | 69,6 | 9,6 | 7,7 | |
| 2 | 57,9 | 16,7 | 13,0 | |
| 3 | 46,6 | 23,5 | 18,3 | |
| 4 | 49,7 | 27,0 | 15,5 | |
| 5,5 | 29,8 | 39,0 | 20,1 | |
| 7,25 | - | 51,8 | 37,5 | 2,4 |

Das Molverhältnis von Tiglinaldehyd zu 2-Methylbutanal betrug laut quantitativer GC-Analyse 1,1 zu 1.

**Beispiel 3**

Entsprechend Beispiel 1 wurde der Reaktor mit 160 g eines $Pd/Pr_2O_3$-Katalysators (0,5 % Pd, 5 % $Pr_2O_3$ auf $\gamma$-$Al_2O_3$ als Träger) beschickt und bei 50°C mit 220 g Ethylacrolein und 6 Liter Wasserstoff/Stunde betrieben. Nach sechs Stunden war praktisch das gesamte Ethylacrolein umgesetzt.

Das Molverhältnis von Tiglinaldehyd zu 2-Methylbutanal betrug laut quantitativer GC-Analyse zu diesem Zeitpunkt 0,77 : 1.

**Beispiel 4**

Wie in Beispiel 1 beschrieben, wurden 125 g Palladium/$CeO_2/Al_2O_3$-Katalysator aktiviert und zunächst 4 Stunden bei 50 ± 2°C, dann 3 Stunden bei 100 ± 2°C mit 280 g n-Hexylacrolein umgesetzt. Sechs Liter Wasserstoff wurden pro Stunde eingeleitet. Nach 7 Stunden war praktisch das gesamte n-Hexylacrolein umgesetzt. Das Molverhältnis von 2-Methyl-2-octenal zu 2-Methyloctanal betrug 0,63 : 1. Durch fraktionierende Destillation von 110 g Reaktionsaustrag wurden 57,3 g 2-Methyloctanal vom Siedepunkt 79°C/30 mbar ($n_D^{20}$ = 1,4200) und 35,7 g 2-Methyl-2-octenal vom Siedepunkt 92°C/30 mbar ($n_D^{20}$ = 1,4555) gewonnen.

**Beispiel 5**

Wie in Beispiel 1 beschrieben, wurden 25 g Palladium/$CeO_2/Al_2O_3$-Katalysator aktiviert und bei 100 ± 2°C 8 Stunden lang mit einer Lösung von 60 g n-Nonylacrolein in 240 g Dioxan umgesetzt. Sechs Liter Wasserstoff wurden pro Stunde eingeleitet. Nach 8 Stunden enthielt das Reaktionsgemisch nur noch 4,5 % n-Nonylacrolein. Das Molverhältnis von 2-Methyl-2-undecenal zu 2-Methylundecanal betrug laut quantitativer GC-Analyse 0,35 : 1.

**Beispiel 6**

In einem 1-l-Hydrierautoklaven wurden 950 ml (670 g) eines Palladium und Cer enthaltenden Katalysators (0,08 % PdO 0,61 % $CeO_2$ auf 4 mm $\gamma$-$Al_2O_3$-Strängen) bei 180°C 24 Stunden lang mit Wasserstoff aktiviert. Dann wurden 24 Stunden lang bei 50°C und 2 bar Wasserstoffdruck 100 ml 2-Ethylacrolein pro Stunde in Rieselfahrweise über den Katalysator gepumpt. Ein Teil der Flüssigkeit wurde zurück über den Katalysator geleitet. Der Innerhalb von 24 Stunden angesammelte Hydrieraustrag besaß folgende Zusammensetzung: 53,6 % Tiglinaldehyd, 29,1 % 2-Methylbutanal und 12,1 % unumgesetztes 2-Ethylacrolein.

**Patentansprüche**

1. Verfahren zur Herstellung von 2-Methyl-2-alkenalen der Formel

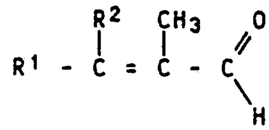

I,

in der $R^1$ und $R^2$ Wasserstoffatome, Alkylreste mit 1 bis 18 Kohlenstoffatomen, die zusätzlich aromatische Reste tragen können, oder aromatische Reste bedeuten, dadurch gekennzeichnet, daß man 2-Alkylacroleine der Formel

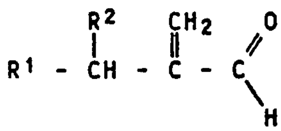

II,

in der $R^1$ und $R^2$ die obengenannte Bedeutung besitzen, in Gegenwart von Katalysatoren, die als aktive Bestandteile Palladium und ein Oxid oder Salz eines Seltenen Erdmetalls enthalten, bei Temperaturen von 20 bis 120°C und Drücken von 1 bis 100 bar mit Wasserstoff behandelt.

2. Verfahren nach Anspruch 1, dadurch gekennzeichnet, daß die aktive Masse des Katalysators zu 2 bis 90 Gew.-% aus Palladium und zu 10 bis 98 Gew.-% aus einem Oxid oder einem Salz eines Seltenen Erdmetalls besteht.

3. Verfahren nach Anspruch 1, dadurch gekennzeichnet, daß man als Katalysatoren Trägerkatalysatoren verwendet, die 0,01 bis 10 Gew.-% Palladium und 0,01 bis 20 Gew.-% eines Oxides oder Salzes eines Seltenen Erdmetalles enthalten.

4. Verfahren nach Anspruch 3, dadurch gekeinzeichnet, daß der Trägerkatalysator als Trägermaterial Aktivkohle, Aluminiumoxid oder Siliciumdioxid enthält.

5. Verfahren nach Anspruch 1, dadurch gekennzeichnet, daß man die Behndlung bei Temperaturen von 50 bis 100°C und Drücken vor 1 bis 20 bar vornimmt.

6. Verfahren nach Anspruch 1 dadurch gekennzeichnet, daß man in Gegenwart eines unter den Reaktionsbedingungen inerten Lösungsmittels arbeitet.

7. Verfahren nach Anspruch 1, dadurch gekennzeichnet, daß in den 2-Alkylacroleinen der Formel II die Reste $R^1$ und $R^2$ Wasserstoffatome oder Alkylreste mit 1 bis 12 C-Atomen bedeuten.

**Claims**

1. A process for the preparation of a 2-methyl-2-alkenal of the formula

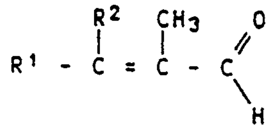

I,

where $R^1$ and $R^2$ are each hydrogen, alkyl of 1 to 18 carbon atoms which may additionally carry aromatic radicals, or an aromatic radical, wherein a 2-alkylacrolein of the formula

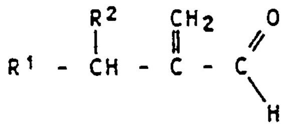

II.

where $R^1$ and $R^2$ have the above meanings, is treated with hydrogen in the presence of a catalyst which contains palladium and an oxide or salt of a rare earth metal as active components, at from 20 to 120°C and under from 1 to 100 bar.

2. A process as claimed in claim 1, wherein the active material of the catalyst consists of from 2 to 90 % by weight of palladium and from 10 to 98 % by weight of an oxide or salt of a rare earth metal.

3. A process as claimed in claim 1, wherein the catalyst used is a supported catalyst which contains from 0.01 to 10 % by weight of palladium and from 0.01 to 20 % by weight of an oxide or salt of a rare earth metal.

4. A process as claimed in claim 3, wherein the supported catalyst contains active carbon, alumina or silica as the carrier.

5. A process as claimed in claim 1, wherein the treatment is carried out at from 50 to 100°C and under from 1 to 20 bar.

6. A process as claimed in claim 1, which is carried out in the presence of a solvent which is inert under the reaction conditions.

7. A process as claimed in claim 1, wherein, in the 2-alkylacrolein of the formula II, $R^1$ and $R^2$ are each hydrogen or alkyl of 1 to 12 carbon atoms.

**Revendications**

1. Procédé de préparation de 2-méthyl-2-alcénals de formule

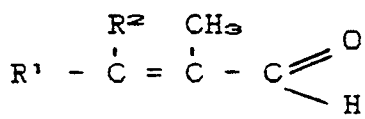

I

dans laquelle $R^1$ et $R^2$ représentent des atomes d'hydrogène, des radicaux alkyle à 1 - 18 atomes de carbone, qui peuvent porter en plus des radicaux aromatiques, ou des radicaux aromatiques, caractérisé en ce qu'on traite par l'hydrogène des 2-alkylacroléines de formule

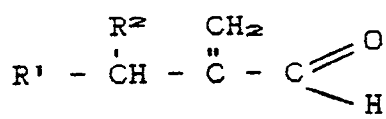

II

dans laquelle $R^1$ et $R^2$ ont les significations données ci-dessus, en présence de catalyseurs qui contiennent, en tant que composants actifs, du palladium et un oxyde ou un sel d'un métal des terres rares, a des températures de 20 à 120°C et sous des pressions de 1 à 100 bars.

2. Procédé selon la revendication 1, caractérisé en ce que la matière active du catalyseur se compose, pour 2 à 90 % en poids, de palladium et, pour 10 à 98 % en poids, d'un oxyde ou d'un sel d'un métal des terres rares.

3. Procédé selon la revendication 1, caractérisé en ce qu'on utilise, comme catalyseurs, des catalyseurs sur support qui contiennent de 0,01 à 10 % en poids de palladium et de 0,01 à 20 % en poids d'un oxyde ou d'un sel d'un métal des terres rares.

4. Procédé selon la revendication 3, caractérisé en ce que le catalyseur sur support contient, en tant que matière de support, un charbon actif, un oxyde d'aluminium ou un bioxyde de silicium.

5. Procédé selon la revendication 1, caractérisé en ce qu'on procède au traitement à des températures de 50 à 100°C et sous des pressions de 1 à 20 bars.

6. Procédé selon la revendication 1, caractérisé en ce qu'on opère en présence d'un solvant inerte dans les conditions de la réaction.

7. Procédé selon la revendication 1, caractérisé en ce que dans les 2-alkylacroléines de formule II, les radicaux $R^1$ et $R^2$ représentent des atomes d'hydrogène ou des radicaux alkyle à 1 - 12 atomes de carbone.